(19) Europäisches Patentamt
European Patent Office
Office européen des brevets

(11) Veröffentlichungsnummer: **0 548 670 A1**

(12) **EUROPÄISCHE PATENTANMELDUNG**

(21) Anmeldenummer: **92120944.1**

(22) Anmeldetag: **09.12.92**

(51) Int. Cl.$^5$: **C07D 263/04**, C07D 263/52, C07F 7/18

(30) Priorität: **20.12.91 DE 4142187**

(43) Veröffentlichungstag der Anmeldung: **30.06.93 Patentblatt 93/26**

(84) Benannte Vertragsstaaten: **AT BE CH DE DK ES FR GB GR IT LI LU MC NL PT SE**

(71) Anmelder: **BAYER AG**

**W-5090 Leverkusen 1 Bayerwerk(DE)**

(72) Erfinder: **Hoppe, Dieter, Dr.**
**Schneiderkamp 17e**
**W-2300 Kiel(DE)**
Erfinder: **Ahrens, Hartmut**
**Holtenauer Strasse 337**
**W-2300 Kiel(DE)**
Erfinder: **Paetow, Mario**
**Fuchsbrgsiedlung 5a**
**W-2401 Warnsdorf(DE)**
Erfinder: **Hintze, Folker**
**Bremerstrasse 6**
**W-2300 Kiel(DE)**

(54) **Heterocyclische Mono- und Dicarbamate.**

(57) Die Erfindung betrifft neue heterocyclische Mono- und Dicarbamate

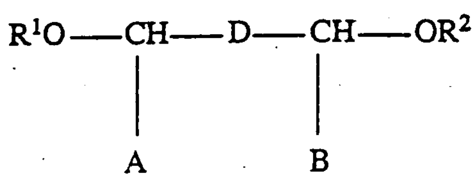

(I),

in welcher
$R^1$ und $R^2$ gleich oder verschieden sind und für einen heterocyclischen Rest der formel

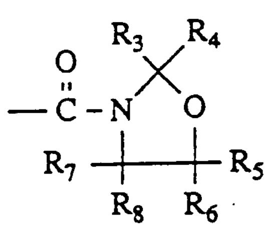

stehen,

worin
$R^3$, $R^4$, $R^5$, $R^6$, $R^7$ und $R^8$ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten, oder jeweils $R^3$ und $R^4$, $R^5$ und $R^6$ und/oder $R^7$ und $R^8$ gemeinsam einen 3- bis 6-gliedrigen, gesättigten Carbocyclus bilden,
oder

EP 0 548 670 A1

$R^1$ für die $(CH_3)_3C\text{-}SI\text{-}(CH_3)_2$-Gruppe steht,
und
$R^2$ die oben angegebene Bedeutung hat,
die wichtige Zwischenprodukte zur Synthese von pharmazeutischen Wirkstoffen, insbesondere zur Synthese von biologisch aktiven Peptiden und Peptid-Mimetika sind, sowie ein neues Verfahren zu ihrer Herstellung.

Die Erfindung betrifft neue heterocyclische Mono- und Dicarbamate, die wichtige Zwischenprodukte zur Synthese von pharmazeutischen Wirkstoffen, insbesondere zur Synthese von biologisch aktiven Peptiden und Peptid-Mimetika sind, sowie ein neues Verfahren zu ihrer Herstellung.

Die Erfindung betrifft Dicarbamate der allgemeinen Formel (I)

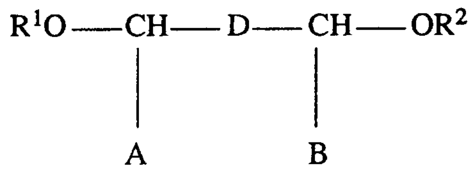

(I),

in welcher

$R^1$ und $R^2$ gleich oder verschieden sind und für einen heterocyclischen Rest der Formel

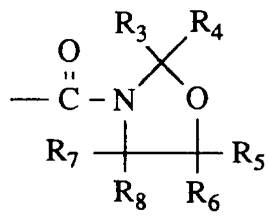

stehen,

worin

$R^3$, $R^4$, $R^5$, $R^6$, $R^7$ und $R^8$ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten, oder jeweils $R^3$ und $R^4$, $R^5$ und $R^6$ und/oder $R^7$ und $R^8$ gemeinsam einen 3- bis 6-gliedrigen, gesättigten Carbocyclus bilden,

oder

$R^1$ für die $(CH_3)_3C\text{-}Si\text{-}(CH_3)_2$-Gruppe steht

und

$R^2$ die oben angegebene Bedeutung hat,

A und B gleich oder verschieden sind und für geradkettiges oder verzweigtes Alkyl oder Alkenyl mit jeweils bis zu 10 Kohlenstoffatomen stehen, die gegebenenfalls bis zu 3-fach gleich oder verschieden durch Hydroxy, Phenyl oder Cycloalkyl mit 3 bis 7 Kohlenstoffatomen oder durch eine Gruppe der Formel $-NR^9R^{10}$, $-HN\text{-}CO\text{-}OR^{11}$, $-SiR^{12}R^{13}R^{14}$ oder $SnR^{12'}R^{13'}R^{14'}$ substituiert sind,

worin

$R^9$ und $R^{10}$ gleich oder verschieden sind und Wasserstoff, geradkettiges oder verzweigtes Alkyl mit bis zu 8 Kohlenstoffatomen, Phenyl oder Benzyl bedeuten,

und

$R^{11}$ geradkettiges oder verzweigtes Alkyl mit bis zu 6 Kohlenstoffatomen bedeutet, das gegebenenfalls durch Phenyl substituiert ist,

$R^{12}$, $R^{12'}$, $R^{13}$, $R^{13'}$, $R^{14}$ und $R^{14'}$ gleich oder verschieden sind und Wasserstoff, geradkettiges oder verzweigtes Alkyl mit bis zu 8 Kohlenstoffatomen oder Phenyl bedeuten,

für geradkettiges oder verzweigtes Acyl mit bis zu 8 Kohlenstoffatomen stehen,

für Cycloalkyl oder Cycloalkenyl mit 3 bis 7 Kohlenstoffatomen stehen, das gegebenenfalls durch Hydroxy substituiert ist,

für Carboxy, Alkoxycarbonyl mit bis zu 4 Kohlenstoffatomen oder für eine Gruppe der Formel $-SiR^{12}R^{13}R^{14}$, $-SnR^{12'}R^{13'}R^{14'}$ oder $R^{15}$-CO

stehen,
worin

$R^{12}$, $R^{12'}$, $R^{13}$, $R^{13'}$, $R^{14}$ und $R^{14'}$ die oben angegebene Bedeutung haben,
und

$R^{15}$ Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel -NH-CO-$OR^{11}$ substituiert sind,
worin
$R^{11}$ die oben angegebene Bedeutung hat,

oder
einer der beiden Substituenten A oder B für Wasserstoff steht,

D für geradkettiges oder verzweigtes Alkyl mit bis zu 15 Kohlenstoffatomen steht, das gegebenenfalls durch Phenyl oder durch die Gruppe der Formel $-NR^9R^{10}$ substituiert ist,
worin

$R^9$ und $R^{10}$ die oben angegebene Bedeutung haben.

Bevorzugt sind Verbindungen der allgemeinen Formel (I),
in welcher

$R^1$ und $R^2$ gleich oder verschieden sind und für einen heterocyclischen Rest der Formel

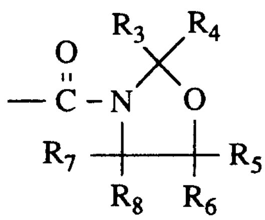

stehen,
worin

$R^3$, $R^4$, $R^5$, $R^6$, $R^7$ und $R^8$ gleich oder verschieden sind und Wasserstoff, geradkettiges oder verzweigtes Alkyl mit bis zu 6 Kohlenstoffatomen, Cyclopropyl, Cyclopentyl oder Cyclohexyl bedeuten, oder $R^3$ und $R^4$, $R^5$ und $R^6$ und/oder $R^7$ und $R^8$ gemeinsam einen Cyclopropyl-, Cyclopentyl- oder Cyclohexylring bilden,

oder

$R^1$ für die $(CH_3)_3C\text{-}Si\text{-}(CH_3)_2$-Gruppe steht

und

$R^2$ die oben angegebene Bedeutung hat,

A und B gleich oder verschieden sind und für geradkettiges oder verzweigtes Alkyl oder Alkenyl mit jeweils bis zu 8 Kohlenstoffatomen stehen, die gegebenenfalls bis zu 3-fach gleich oder verschieden durch Hydroxy, Phenyl, Cyclobutyl, Cyclopentyl, Cyclohexyl oder durch eine Gruppe der Formel $-NR^9R^{10}$, -NH-CO-$OR^{11}$, $-SiR^{12}R^{13}R^{14}$ oder $-SnR^{12'}R^{13'}R^{14'}$ substituiert sind,
worin

$R^9$ und $R^{10}$ gleich oder verschieden sind und Wasserstoff, geradkettiges oder verzweigtes Alkyl mit bis zu 6 Kohlenstoffatomen, Phenyl oder Benzyl bedeuten,

$R^{11}$ geradkettiges oder verzweigtes Alkyl mit bis zu 4 Kohlenstoffatomen bedeutet, das gegebenenfalls durch Phenyl substituiert ist,

$R^{12}$, $R^{12'}$, $R^{13}$, $R^{13'}$, $R^{14}$ und $R^{14'}$ gleich oder verschieden sind und geradkettiges oder verzweigtes Alkyl mit bis zu 4 Kohlenstoffatomen oder Phenyl bedeuten,

für geradkettiges oder verzweigtes Acyl mit bis zu 6 Kohlenstoffatomen stehen,

für Cyclobutyl oder Cyclohexyl stehen, die gegebenenfalls durch

Hydroxy substituiert sind,
für Carboxy, Alkoxycarbonyl mit bis zu 3 Kohlenstoffatomen oder für eine Gruppe der Formel $-SiR^{12}R^{13}R^{14}$, $-SnR^{12'}R^{13'}R^{14'}$ oder $R^{15}$-CO- steht,
worin

$R^{12}$, $R^{12'}$, $R^{13}$, $R^{13'}$, $R^{14}$ und $R^{14'}$ die oben angegebene Bedeutung haben
und

$R^{15}$ Wasserstoff oder geradkettiges oder verzweigtes Alkyl oder Alkenyl mit jeweils bis zu 6 Kohlenstoffatomen bedeutet, die gegebenenfalls durch Phenyl oder durch die Gruppe der Formel $-NH-CO-OR^{11}$ substituiert sind,
worin

$R^{11}$ die oben angegebene Bedeutung hat,

oder
einer der beiden Substituenten A oder B für Wasserstoff steht,

D für geradkettiges oder verzweigtes Alkyl mit bis zu 14 Kohlenstoffatomen steht, das gegebenenfalls durch Phenyl oder durch die Gruppe der Formel $-NR^9R^{10}$ substituiert ist,
worin

$R^9$ und $R^{10}$ die oben angegebene Bedeutung haben.

Besonders bevorzugt sind Verbindungen der allgemeinen Formel (I),
in welcher

$R^1$ und $R^2$ gleich oder verschieden sind und für einen heterocyclischen Rest der Formel

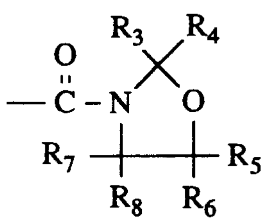

stehen,
worin

$R^3$, $R^4$, $R^5$, $R^6$, $R^7$ und $R^8$ gleich oder verschieden sind und Wasserstoff, geradkettiges oder verzweigtes Alkyl mit bis zu 4 Kohlenstoffatomen, Cyclopropyl oder Cyclopentyl bedeuten, oder $R^3$ und $R^4$, $R^5$ und $R^6$ und/oder $R^7$ und $R^8$ gemeinsam einen Cyclopropyl-, Cyclopentyl- oder Cyclohexylring bilden,

oder

$R^1$ für die $(CH_3)_3C-Si(CH_3)_2$-Gruppe steht

und

$R^2$ die oben angegebene Bedeutung hat,

A und B gleich oder verschieden sind und für geradkettiges oder verzweigtes Alkyl oder Alkenyl mit jeweils bis zu 7 Kohlenstoffatomen stehen, die gegebenenfalls bis zu 3-fach gleich oder verschieden durch Hydroxy, Phenyl, Cyclobutyl, Cyclohexyl oder durch eine Gruppe der Formel $-NR^9R^{10}$, $-NHCO-OR^{11}$, $-SiR^{12}R^{13}R^{14}$ oder $-SnR^{12'}R^{13'}R^{14'}$ substituiert sind,
worin

$R^9$ und $R^{10}$ gleich oder verschieden sind und Wasserstoff, geradkettiges oder verzweigtes Alkyl mit bis zu 4 Kohlenstoffatomen, Phenyl oder Benzyl bedeuten,

$R^{11}$ Methyl oder Ethyl bedeutet, die gegebenenfalls durch Phenyl substituiert sind,

$R^{12}$, $R^{12'}$, $R^{13}$, $R^{13'}$, $R^{14}$ und $R^{14'}$ für Methyl oder Phenyl stehen,
für geradkettiges oder verzweigtes Acyl mit bis zu 4 Kohlenstoffatomen stehen,

für Cyclobutyl oder Cyclohexyl stehen, die gegebenenfalls durch Hydroxy substituiert sind,
für Carboxy, Alkoxycarbonyl mit bis zu 3 Kohlenstoffatomen oder für eine Gruppe der Formel $-SiR^{12}R^{13}R^{14}$, $-SnR^{12'}R^{13'}R^{14'}$ oder $R^{15}$-CO- stehen,
worin

$R^{12}$, $R^{12'}$, $R^{13}$, $R^{13'}$, $R^{14}$ und $R^{14'}$ die oben angegebene Bedeutung haben
und

$R^{15}$ Wasserstoff oder geradkettiges oder verzweigtes Alkyl oder Alkenyl mit jeweils bis zu 4 Kohlenstoffatomen bedeutet, die gegebenenfalls durch Phenyl oder durch die Gruppe der Formel $-NH-CO-OR^{11}$ substituiert sind,
worin

$R^{11}$ die oben angegebene Bedeutung hat,

oder
einer der beiden Substituenten A oder B für Wasserstoff steht,

D für geradkettiges oder verzweigtes Alkyl mit bis zu 12 Kohlenstoffatomen steht, das gegebenenfalls durch Phenyl oder durch die Gruppe der Formel $-NR^9R^{10}$ substituiert ist,
worin

$R^9$ und $R^{10}$ die oben angegebene Bedeutung haben.

Außerdem wurde ein neues Verfahren zur Herstellung der erfindungsgemäßen Verbindungen der allgemeinen Formel (I) gefunden, dadurch gekennzeichnet, daß man

Verbindungen der allgemeinen Formel (II)

$R^1O-H_2C-D-H_2C-OR^2$ (II),

in welcher
$R^1$, $R^2$ und D die oben angegebene Bedeutung haben,
in inerten Lösemitteln, in Anwesenheit einer selektiven Base, vorzugsweise sec.-Butyllithium und eines chelatbildenden Diamins (im folgenden mit E bezeichnet) zunächst zu den Carbanion-Komplexverbindungen der allgemeinen Formel (III)

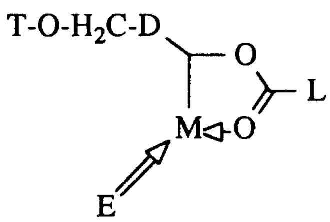

(III),

in welcher

D die oben angegebene Bedeutung hat

und

T für einen der oben aufgeführten Substituenten $R^1$ oder $R^2$ steht,

E für ein chelatbildendes Diamin, vorzugsweise für (-)-Spartein, steht,

M für ein Lithium-, Magnesium-, Titan-, Zirkonium- oder Kaliumatom, vorzugsweise für ein Lithiumatom, steht

und

L für den oben angegebenen heterocyclischen Rest

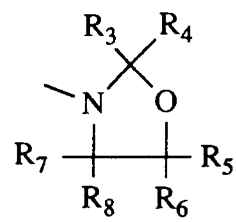

steht,
worin

$R^3$, $R^4$, $R^5$, $R^6$, $R^7$ und $R^8$ die oben angegebene Bedeutung haben,

enantioselektiv deprotoniert; anschließend mit Elektrophilen der allgemeinen Formeln (IV) oder (V)

W-X (IV)

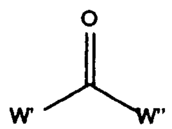
(V),

in welcher

W für einen der oben aufgeführten Substituenten A oder B steht,

X für Halogen, für $C_1$-$C_4$-Alkoxy oder für eine typische Abgangsgruppe, vorzugsweise für Chlor, steht,

und

W' und W'' gleich oder verschieden sind und für einen unter A aufgeführten chemisch sinnvollen Rest stehen,

oder mit $CO_2$ in die Verbindungen der allgemeinen Formel (Ia)

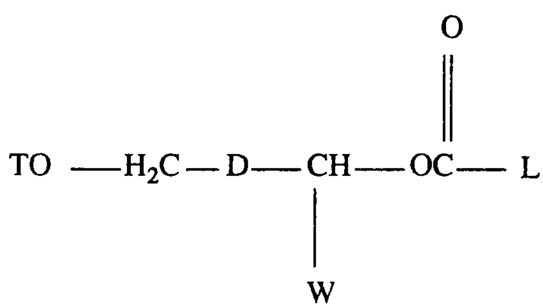
(Ia),

in welcher

T, W, L und D die oben angegebene Bedeutung haben,

umsetzt und im Fall, daß $R^1 \neq (CH_3)_3C\text{-}Si(CH_3)_2$, gegebenenfalls dieses Verfahren zur Einführung des zweiten Substituenten (A oder B) über den Carbanion-Komplex der Formel (VI)

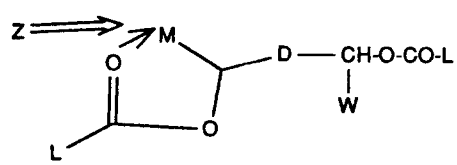
(VI),

in welcher

M, L und W die oben angegebene Bedeutung haben,

und

Z entweder für den chiralen Komplexbildner (-)-Spartein oder für den achiralen Komplexbildner TMEDA steht,

unter stereochemischer Lenkung wiederholt.

Das erfindungsgemäße Verfahren kann durch folgende Formelschemen beispielhaft erläutert werden:

Formelschema 1

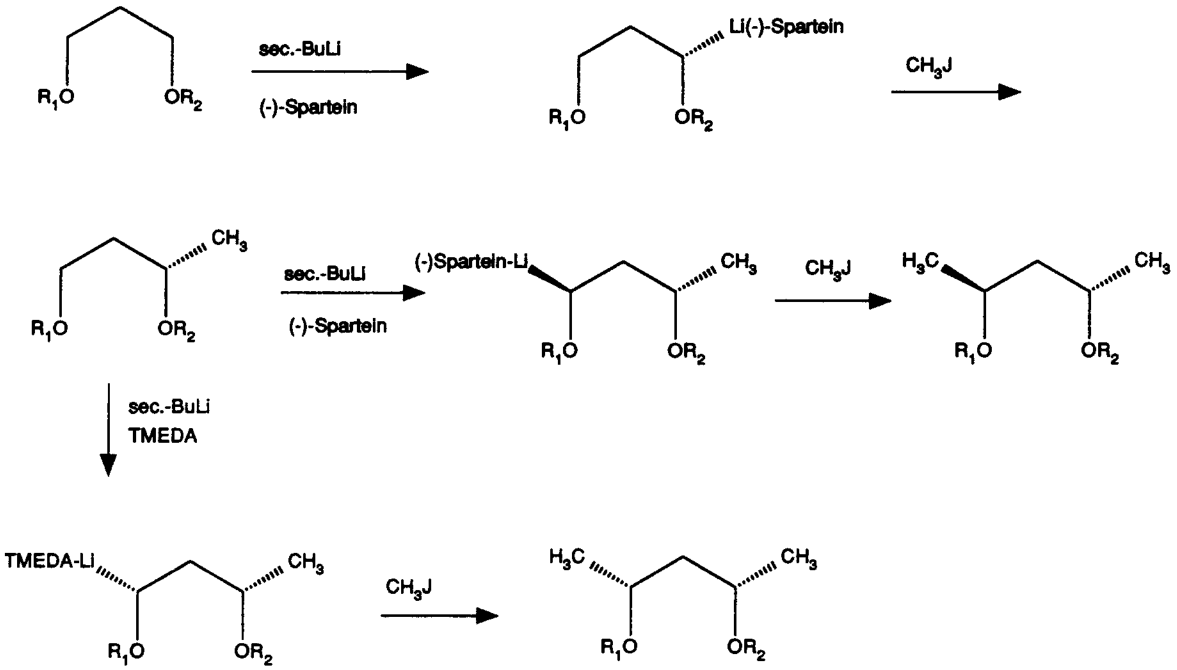


b = $R^1$ / $R^2$ = 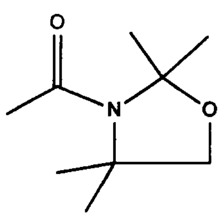

**Formelschema 2**

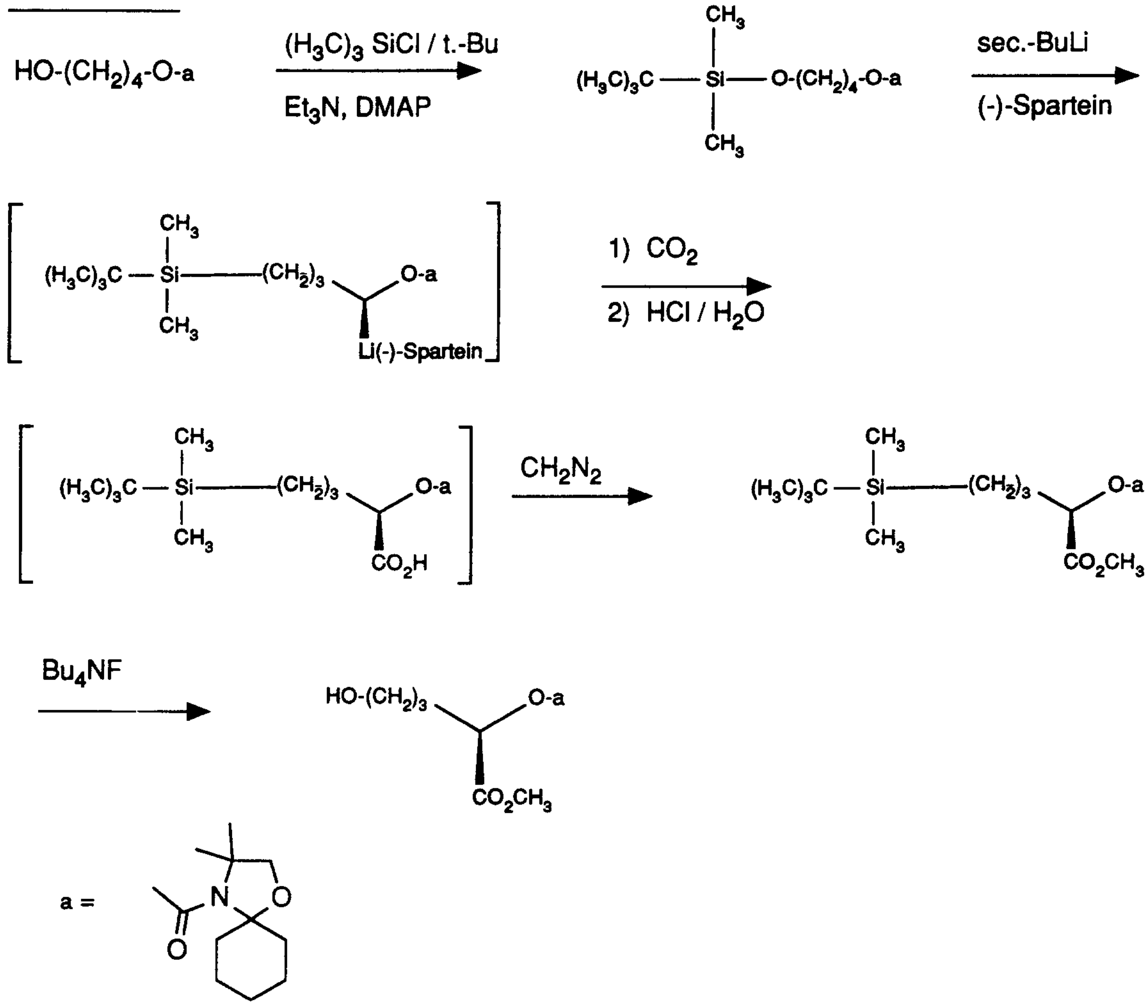


Überraschenderweise liefert das erfindungsgemäße Verfahren die erfindungsgemäßen Verbindungen in sehr guten Ausbeute.

Bei Kenntnis des Standes der Technik zeichnet sich das oben angegebene Verfahren durch mehrere Vorteile aus. Überraschenderweise erfolgt weder eine Zersetzung unter Wanderung einer der beiden Gruppen $R^1$ oder $R^2$ noch eine Cyclisierung bei tiefen Temperaturen. Außerdem kann insbesondere bei der zweiten elektrophilen Substitution durch Einsatz eines chiralen oder achiralen Komplexbildners eine stereochemische Lenkung in guten Ausbeuten erreicht werden. Unter Bezug auf das stereochemische Zentrum in ω-Position wird mit dem chiralen (-)-Spartein eine externe chirale Induktion unter Si-Deprotonierung, im Fall des achiralen TMEDA eine interne chirale Induktion hervorgerufen, wobei insbesondere die interne chirale Induktion von den strukturellen Gegebenheiten beeinflußt wird.

Der oben aufgeführte Begriff Abgangsgruppe steht im allgemeinen für Chlor, Brom, Jod, Tosylat, Mesylat oder dem Rest $-OSO_2CF_3$.

Als Lösemittel für die Deprotonierung eignen sich bevorzugt inerte organische Lösemittel wie Kohlenwasserstoffe wie Hexan, Pentan, Ligroin oder Toluol, und Ether, beispielsweise Tetrahydrofuran, Diethylether, Dioxan, Dimethoxyethan, Diglyme, Triglyme oder tert.-Butylmethylether. Besonders bevorzugt sind Tetrahydrofuran und Diethylether.

Die Deprotonierung erfolgt in einem Temperaturbereich von - 100 °C bis Raumtemperatur, vorzugsweise bei ca. -78 °C bis 0 °C.

Die Deprotonierung kann sowohl bei Normaldruck als auch bei erhöhtem oder erniedrigtem Druck (z.b. 0,5 bis 2 bar), vorzugsweise bei Normaldruck durchgeführt werden.

Als selektive Basen eignen sich Alkyllithiumverbindungen mit bis zu 6 C-Atomen in der Alkylgruppe, vorzugsweise n-Butyllithium oder sec.-Butyllithium.

Die Base wird in einer Menge von 0,5 bis 5 mol, vorzugsweise in stöchiometrischen Mengen, bezogen auf 1 mol der Verbindungen der allgemeinen Formel (II), eingesetzt.

Die elektrophile Substitution erfolgt ebenfalls in den oben aufgeführten Lösemitteln, bevorzugt in Tetrahydrofuran bei Normaldruck.

Die elektrophile Substitution wird in einem Temperaturbereich von ca. - 100 °C bis +40 °C, vorzugsweise im Bereich von -78 °C bis Raumtemperatur durchgeführt.

Die Verbindungen der allgemeinen Formel (II) sind neu und können hergestellt werden, indem man Verbindungen der allgemeinen Formel (VI)

$HO-H_2C-D-CH_2-OH$ (VI),

in welcher
D die oben angegebene Bedeutung hat,
mit aktivierten Heterocyclen der allgemeinen Formel (VII)

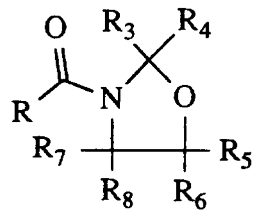

(VII),

in welcher
$R^3$,$R^4$,$R^5$,$R^6$,$R^7$ und $R^8$ die oben angegebene Bedeutung haben
und
R für Halogen, vorzugsweise für Chlor steht,
in einem der oben aufgeführten Lösemittel, vorzugsweise in Ethern, gegebenenfalls in Anwesenheit einer der oben aufgeführten Basen umsetzt.

Die Verbindungen der allgemeinen Formeln (Ia) und (III) sind ebenfalls neu und können nach dem oben angegebenen Verfahren hergestellt werden.

Die Verbindungen der allgemeinen Formeln (IV) und (V) und die Verbindungen der allgemeinen Formeln (VI) und (VII) sind bekannt oder können nach üblicher Methode hergestellt werden.

Die Abspaltung der Silylschutzgruppen erfolgt mit Ammoniumfluoriden, insbesondere mit $(C_4H_9)N^+F^-$.

Als Säuren eignen sich starke anorganische Säuren und organische Sulfon-oder Carbonsäuren wie beispielsweise Methansulfonsäure, Ethansulfonsäure, Benzolsulfonsäure, Toluolsulfonsäure, Essigsäure oder Propionsäure.

Als Basen eignen sich sich Alkali- und Erdalkalihydroxide wie beispielsweise Natrium-, Kalium- oder Bariumhydroxid. Bevorzugt ist Bariumhydroxid.

Die Säuren und Basen werden in einer Menge von 0,01 bis 10 mol, vorzugsweise 1 mol, bezogen auf 1 mol der Verbindungen der allgemeinen Formel (I) eingesetzt.

Die Abspaltung der Carbamatschutzgruppen erfolgt bei Normaldruck in einem Temperaturbereich von 0 °C bis +130 °C, vorzugsweise von +20 °C bis +100 °C.

Die erfindungsgemäßen Verbindungen der allgemeinen Formel (I) zeichnen sich einerseits, wie oben detailliert beschrieben, durch die leichte Einführung von Elektrophilen unter stereochemischer Lenkung aus; andererseits sind die Schutzgruppen $R^1$/$R^2$ unter Freisetzung der entsprechenden Hydroxyfunktion extrem leicht abspaltbar oder können unter Einsatz von Säuren in guten Ausbeuten direkt zu Lactonen cyclisiert werden. Insbesondere im Fall der Silylschutzgruppe wird somit ein Zugang zur Freisetzung von ω-OH-Funktion ermöglicht.

Die erfindungsgemäßen Verbindungen sind somit wertvolle Zwischenprodukte zur Herstellung von bekannten offenkettigen Hydroxyverbindungen bzw. Lactonen, die wiederum eine wichtige Rolle zur Synthese von Peptiden und Peptid-Mimetika übernehmen.

Lösemittel:

1) Ether: Pentan = 1:1
2) Chloroform

3) Pentan
4) Ether: Pentan = 1:4
5) Ether: Pentan = 1:2

Im folgenden bedeutet unter den Definitionen $R^1/R^2$ a und b folgende Reste:

a =

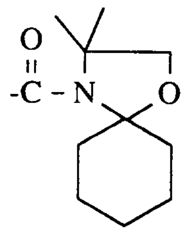

b =

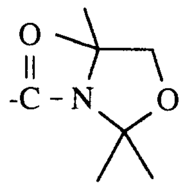

Ausgangsverbindungen

Beispiel 1

2,2-Dimethylpropan-1,3-diyl-bis(2,2,4,4-tetramethyl-1,3-oxazolidin-3-carboxylat)

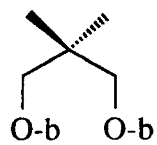

Zu einer Suspension aus 1,95 g (65 mmol) Natriumhydrid (80%ig in Parrafinöl) in 50 ml THF werden 3,12 g (30 mmol) 2,2-Dimethylpropan-1,3-diol langsam zugegeben und 2,5 h bei Raumtemperatur gerührt. Man injiziert 12,0 g (63 mmol) 2,2,4,4-Tetramethyl-1,3-oxazolidin-3-carbonylchlorid in 20 ml THF und rührt 48 h bei Raumtemperatur. Die Reaktionslösung wird auf 100 ml 2 N Salzsäure gegossen, die Phasen werden getrennt und die wäßrige Phase zweimal mit 50 ml Ether extrahiert. Die vereinigten organischen Phasen werden mit 50 ml ges. Natriumhydrogencarbonatlösung gewaschen, über Natriumsulfat getrocknet und das Lösemittel im Vakuum entfernt. Das Rohprodukt wird flüssigkeitschromatographisch an 700 g Kieselgel mit Ether/Pentan (1:2) gereinigt.
Ausbeute: 8,94 g (72% der Theorie)
$R_f$ = 0,34 g (Kieselgel Ether/Pentan 1:1)
In Analogie zur Vorschrift des Beispiels 1 werden die in Tabelle 1 aufgeführten Beispiele hergestellt:

Tabelle I:

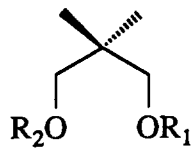

| Bsp.-Nr. | $R^1$ | $R^2$ | $R_f$ (*LM) | Ausbeute (% d.Th.) | F°C |
|---|---|---|---|---|---|
| II | 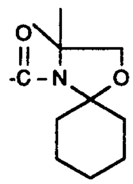 | 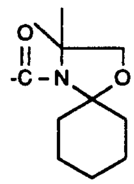 | 0,56[1] | 81 | 114 |

Beispiel III

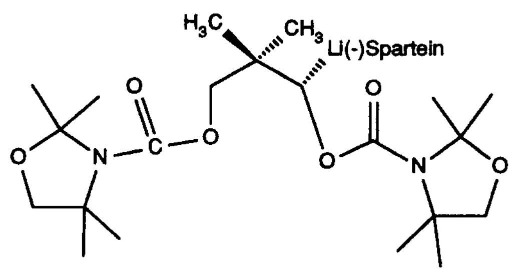

1,51 g (3,7 mmol) der Verbindung aus Beispiel I werden in 10 ml Ether gelöst und tropfenweise zu einer Lösung aus 1,54 g (6,6 mmol) (-)-Spartein und 5 ml (6,7 mmol) sec.-Butyllithium (als 1,3 N Lösung in Cyclohexan/Isopentan) in 15 ml Ether bei -78 ° C zugegeben.

In Analogie zur Vorschrift der Beispiele I, II und III werden die in Tabelle II aufgeführten Verbindungen hergestellt:

Tabelle II

| $R^1$-O-$CH_2$-D-$CH_2$-O-$R^2$ | | | | | |
|---|---|---|---|---|---|
| Bsp.-Nr. | D | $R^1/R^2$ | Ausbeute (% d.Th.) | Schmp. (° C) | $R_f$/Solv. |
| IV | $CH_2$ | a | 85 | 95[1)] | 0,47[1)] |
| V | $CH_2$ | b | 90 | 64 | 0.34[1)] |
| VI | $CH(CH_3)$ | b | 68 | | |
| VII | $CH(C_6H_5)$ | b | | | |
| VIII | $(CH_2)_2$ | a | 59 | 127[2)] | 0,39[1)] |
| IX | $(CH_2)_2$ | b | 82 | 108[3)] | 0,26[1)] |
| X | $(CH_2)_{10}$ | b | 94 | 38 | 0,55[1)] |

Beispiel XI

(4-Hydroxybutyl)-3,3-dimethyl-1-oxa-4-azaspiro[4,5]decan-4-carboxylat

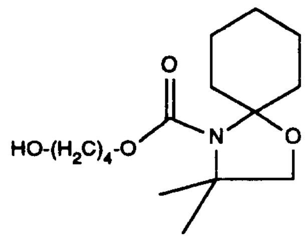

2,62 g (87,3 mmol, 80%ig) Natriumhydrid in Paraffinöl werden in 80 ml wasserfreiem THF suspendiert. Hierzu wird eine Lösung von 26,78 g (297,5 mmol) 4-Butandiol in 20 ml THF langsam hinzugegeben. Nach 30 min. Rühren bei Raumtemperatur werden 15,50 g (67,0 mmol) 3,3-Dimethyl-1-oxa-4-azaspiro[4,5]-decan-4-carbonylchlorid in 50 ml THF eingetropft. Nach 14 h Sieden unter Rückfluß wird das braune Reaktionsgemisch auf 80 ml 2 N HCl und 50 ml Ether gegossen. Nach der Phasentrennung wird die wäßrige Phase dreimal mit je 50 ml Ether extrahiert und die vereinigten Etherlösungen über Natriumhydrogencarbonat/Natriumsulfat (1:2) entsäuert und getrocknet. Anschließend wird das Lösemittel im Vakuum entfernt und das Rohprodukt an Kieselgel (Ether/Pentan = 5:1) gereinigt.
Man erhält 11,47 g (86%) der Titelverbindung als gelbes Öl.
$R_f$ = 0,07 (Ether/Pentan 1:1)

Beispiel XII

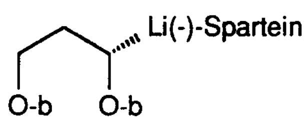

1,93 g (5 mmol) der Verbindung aus Beispiel V werden in 7 ml Ether gelöst und tropfenweise zu einer Lösung aus 1,75 g (7,5 mmol) (-)-Spartein und 5,6 ml (7,6 mmol) sec.-Butyllithium (als ca. 1,3 N Lösung in Cyclohexan/Isopentan) in 10 ml Ether bei -78 °C gegeben.

Beispiel XIII

[4-(tert.Butyldimethylsilyloxy)butyl]-3,3-dimethyl-1-oxa-4-azaspiro[4,5]decan-4-carboxylat

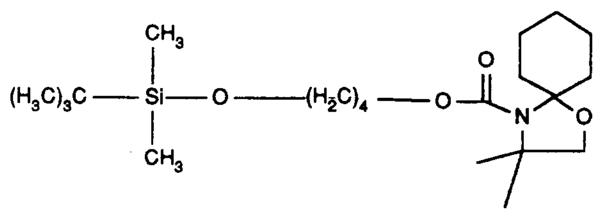

Eine Lösung von 5,40 g (35,6 mmol) tert.Butyldimethylsilylchlorid, 2,57 g (25,5 mmol) Triethylamin und 1,27 g (10,2 mmol) 4-N,N-Dimethylaminopyridin in 150 ml wasserfreiem Dichlormethan wird zu 9,07 g (31,8 mmol) der Verbindung aus Beispiel XI injiziert. Nach 16 h Sieden unter Rückfluß wurde das Gemisch mit 400 ml Ether verdünnt, wobei das dabei ausgefallene Salz nach Zugabe von 150 ml 2 N HCl wieder in Lösung ging. Die organische Phase wird mit gesättigter Natriumhydrogencarbonatlösung entsäuert und über Magnesiumsulfat getrocknet. Nach der Entfernung des Lösemittels im Vakuum wird der Rückstand an

Kieselgel (Ether/Pentan 1:3) chromatographiert und ergibt 12,38 g (98%) der Titelverbindung als schwach gelbliches Öl.
$R_f$ = 0,56 (Ether/Pentan 1:1)
In Analogie zur Vorschrift des Beispiels XIII werden die in Tabelle III aufgeführten Verbindungen hergestellt:

Tabelle III

| * TBDMSO-$CH_2$-D-$CH_2$-$OR^1$ | | | | | |
|---|---|---|---|---|---|
| Bsp.-Nr. | D | $R^1$ | Ausbeute (% d.Th.) | Schmp. (°C) | $R_f$/ Solv. |
| XIV | $-CH_2-$ | b | 96 | Öl | 0,68 [1)] |
| XV | $-(CH_2)_2-$ | a | 98 | Öl | 0,56 [1)] |
| XVI | $-(CH_2)_{10}-$ | b | 97 | Öl | 0,68 [1)] |

* TBDMSO = ω-(tert.Butyldimethylsilyloxy)alkylcarbamat

In Analogie zu den Vorschriften der Beispiele I, II und III werden die in Tabelle IV aufgeführten Verbindungen hergestellt.

Tabelle IV:

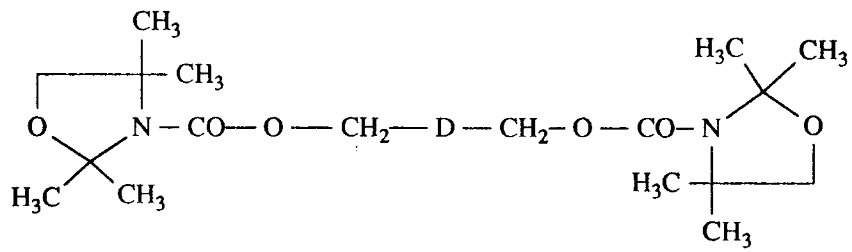

| Bsp.Nr. | D | Drehwert $[\alpha]_D^{20}$ | $F°C/R_f$* | Ausbeute (% d. Th.) | Ausgangsverbindungen |
|---|---|---|---|---|---|
| XVII | $-CH_2-CH$ $N(CH_2-C_6H_5)_2$ | -40,94° ($CHCl_3$) (c = 1) | 0,35[1)] | 71 | $HO-CH_2-CH-(CH_2)_2OH$ $N(CH_2-C_6H_5)_2$ + 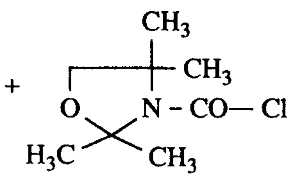 |
| XVIII | $-CH-(CH_2)_3-CH_3$ | | Öl, 0,45[1)] | 87 | $CH_3$ $(CH_2)_3$ $HO-CH_2-CH-CH_2-OH$ + 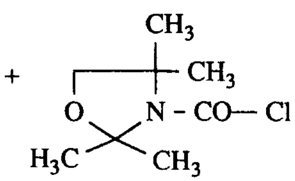 |
| XIX | $-CH-(CH_2)_3-CH_3$ | | | | |
| XX | $-(CH_2)_2$ | | | | |

Herstellungsbeispiele

Beispiel 1

Butan-1,3-diyl-bis(2,2,4,4-tetramethyl-1,3-oxazolidin-3-carboxylat)

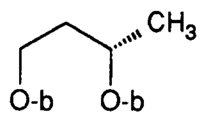

Die Reaktionslösung aus Beispiel XII wird 2,5 h gerührt, mit 1,42 g (4 mmol) Methyliodid versetzt und 3 h nachgerührt. Die auf Raumtemperatur erwärmte Reaktionsmischung wird auf 15 ml 2 N Salzsäure gegossen, die organische Phase abgetrennt und die wäßrige Phase dreimal mit 20 ml Ether extrahiert. Die vereinigten organischen Phasen werden mit 10 ml gesättigter Natriumhydrogencarbonat-Lösung gewaschen, über Natriumsulfat getrocknet und das Lösemittel im Vakuum entfernt. Das Rohprodukt wird chromatographisch an Kieselgel mit Ether / Pentan (1:4) gereinigt. Man erhält 1,67 g (83%) der Titelverbindung als farblosen Feststoff.
$R_f$ = 0,43 (Kieselgel Ether/Pentan 1:1)
Schmp.: 49-52 °C

Beispiel 2

(2R, 4S)-Pentansäure-2,4-diyl-bis(2,2,4,4-tetramethyl-1,3-oxazolidin-3-carboxylat

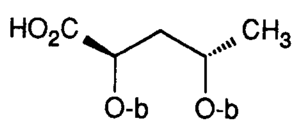

Zu einer Lösung aus 0,53 g (2,25 mmol) (-)-Spartein und 1,7 ml (2,3 mmol) sec.-Butyllithium (als ca. 1,3 N Lösung in Cyclohexan/Isopentan) in 10 ml Ether werden bei -78 °C 0,60 g (1,5 mmol) der Verbindung aus Beispiel 1 in 7 ml Ether langsam zugetropft und 3 h gerührt. Über eine Kanüle wird überschüssiges Kohlendioxid eingeleitet und die Lösung 16 h nachgerührt, wobei sie langsam auf Raumtemperatur erwärmt wird. Die Reaktionsmischung wird auf 20 ml 2 N Salzsäure gegossen, die organische Phase abgetrennt und die wäßrige Phase viermal mit 15 ml Ether extrahiert. Die vereinigten organischen Phasen werden über Natriumsulfat getrocknet und das Lösemittel im Vakuum entfernt. Die Titelverbindung kann chromatographisch an Kieselgel mit Ether/Pentan (1:1) gereinigt werden.
$R_f$ = 0,15 (Kieselgel Ether/Pentan 1:1)
Schmp.: 111 °C

In Analogie zur Vorschrift des Beispiels 1 werden die in Tabelle 1 aufgeführten Beispiele hergestellt:

Tabelle 1:

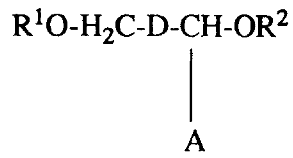

| Bsp.-Nr. | D | A | $R^1/R^2$ | Base/Amin | Ausbeute (% d.Th.) | Schmp. (°C) | Konfiguration / ee |
|---|---|---|---|---|---|---|---|
| 3 | $CH_2$ | $-CH_3$ | a | s-BuLi/(-)-Sp. | 85 | 77 | S / >98 |
| 4 | $CH_2$ | $(CH_3)_3Si-$ | a | s-BuLi/(-)-Sp. | 78 | Öl | S |
| 5 | $CH_2$ | $-CO_2H$ | a | s-BuLi/TMEDA | 68 | 59 | R |
| 6 | $CH_2$ | $C_6H_5-CH_2-$ | b | s-BuLi/(-)-Sp. | 58 | Öl | S |
| 7 | $CH_2$ | $CH_2=CH-CH_2$ | b | s-BuLi/(-)-Sp. | 69 | Öl | S |
| 8 | $CH_2$ | $-CH_3$ | b | s-BuLi/(-)-Sp. | 83 | 50 | |
| 9 | $CH(CH_3)$ | $-CH_3$ | b | s-BuLi/(-)-Sp- | 58 | Öl | Diast. / 3:1 |
| 10 | $CH(CH_3)$ | $(CH_3)_3Si-$ | b | s-BuLi/(-)-Sp. | | | Diast. / 3:1 |
| 11 | $CH(C_6H_5)$ | $-CH_3$ | b | s-BuLi(-)-Sp. | 35 | Öl | Diast. |
| 12 | $C(CH_3)_2$ | $(H_3C)_3Sn-$ | a | s-BuLi/TMEDA | 61 | Öl | Racemat |
| 13 | $C(CH_3)_2$ | $CO_2CH_3$ | a | s-BuLi/TMEDA | 62 | Öl | Racemat |
| 14 | $C(CH_3)_2$ | $CO_2H$ | a | s-BuLi/TMEDA | 72 | 68 | Racemat |

Fortsetzung

| Bsp.-Nr. | D | A | $R^1/R^2$ | Base/Amin | Ausbeute (% d.Th.) | Schmp. (°C) | Konfiguration / ee |
|---|---|---|---|---|---|---|---|
| 15 | $C(CH_3)_2$ | $CO_2H$ | b | s-BuLi/(-)-Sp. | 85 | Öl | R / > 96 |
| 16 | $CH_2$ | -CH=O | b | s-BuLi/(-)-Sp. | 71 | Öl | R |
| 17 | $CH-C_6H_5$ | $-CO_2H$ | b | s-BuLi/(-)-Sp. | 81 | | |
| 18 | $(CH_2)_{10}$ | $-CH_3$ | b | s-BuLi/TMEDA | 29 | Öl | S / Racemat |
| 19 | $(CH_2)_{10}$ | $-CO_2CH_3$ | b | s-BuLi/(-)-Sp. | 24 | Öl | S |
| 20 | $CH_2$ | $(CH_3)_3Si-$ | b | s-BuLi/TMEDA | 73 | Öl | Racemat |

Beispiel 21

(2S, 4S)-Pentan-2,4-diyl-bis(3,3-dimethyl-1-oxa-4-azaspiro[4,5]decan-4-carboxylat

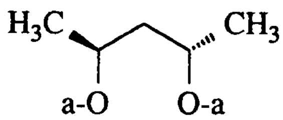

Zu einer Lösung aus 195 mg (0,84 mmol) (-)-Spartein und 0,9 ml (1,1 mmol) sec.-Butyllithium (als ca. 1,3 N Lösung in Cyclohexan/Isopentan) in 10 ml Ether werden bei -78°C 260 mg (0,55 mmol) der Verbindung aus Beispiel 3 in 5 ml Ether zugetropft, 2,5 h gerührt und 280 mg (2 mmol) Methyliodid injiziert. Die Lösung wird 16 h nachgerührt und langsam auf Raumtemperatur erwärmt. Die Reaktionsmischung wird auf 10 ml 2 N Salzsäure gegossen, die organische Phase abgetrennt und die wäßrige Phase dreimal mit 15 ml Ether extrahiert. Die vereinigten organischen Phasen werden mit 10 ml gesättigter Natriumhydrogencarbonatlösung gewaschen, über Natriumsulfat getrocknet und das Lösemittel im Vakuum entfernt. Das Rohprodukt wird flüssigkeitschromatographisch an Kieselgel mit Ether/Pentan (1:4) gereinigt. Man erhält 182 mg (67%) der Titelverbindung mit einer Diastereomerenreinheit von 94% ds und einer optischen Reinheit von 98% ee.

$R_f$ = 0,66 (Kieselgel Ether/Pentan 1:1)

Schmp.: 143°C

Beispiel 22

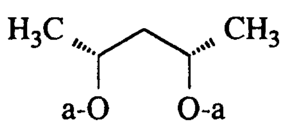

In Analogie zur Vorschrift des Beispiels 21 erhält man durch Verwendung von TMEDA anstelle von (-)-Spartein 65% der Mesoverbindung mit einer Diastereomerenreinheit von 96% ds.

$R_f$ = 0,66 (Kieselgel Ether/Pentan 1:1)

In Analogie zu den Vorschriften der Beispiele 2, 21 und 22 erhält man die in der Tabelle 2 aufgeführten Verbindungen:

Tabelle 2:

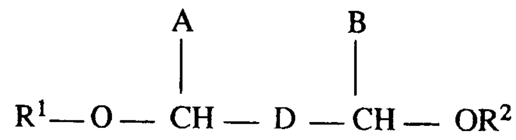

| Bsp.-Nr. | D | A | B | $R^1/R^2$ | Base/Amin | Ausbeute (% d.Th.) | Schmp. (°C) | Konfiguration / ee |
|---|---|---|---|---|---|---|---|---|
| 23 | $-CH_2$ | $-CH_3$ | $-CH_3$ | a | s-BuLi/(-)-Sp. | 35 | 143 | S,S / >99 |
| 24 | $-CH_2$ | $-CH_3$ | $-CH_3$ | a | s-BuLi/TMEDA | | | R, S / 96 |
| 25 | $-CH_2$ | $-CH_3$ | $-CO_2H$ | b | s-BuLi/(-)-Sp. | 60 | 111 | R, S / 95 |
| 26 | $-(CH_2)_2$ | $-CH_3$ | $-CH_3$ | a | s-BuLi/(-)-Sp. | 20 | Öl | S, S |
| 27 | $-(CH_2)_2$ | $-CH_3$ | $-CH_3$ | b | s.-BuLi/(-)-Sp. | 51 | 55 | S, S / 98 |
| 28 | $-(CH_2)_2$ | $-CH_3$ | $-CH_3$ | b | s-BuLi/TMEDA | | 54 | S, S / 95 |
| 29 | $-(CH_2)_2$ | $-CH_3$ | $-CO_2CH_3$ | b | s-BuLi/(-)-Sp. | 33 | 60 | R, S |
| 30 | $-(CH_2)_{10}$ | $-CH_3$ | $-CH_3$ | b | s-BuLi/(-)-Sp. | 13 | Öl | S, S |
| 31 | $-(CH_2)_{10}$ | $-CH_3$ | $-CO_2CH_3$ | b | s-BuLi/(-)-Sp. | 22 | Öl | R, S |

Beispiel 32

(-)-(R)-4-(tert.Butyldimethylsilyloxy)-2-[(3,3-dimethyl-1-oxa-4-azaspiro[4,5]dec-4-ylcarbonyl)oxy]-pentansäuremethylester

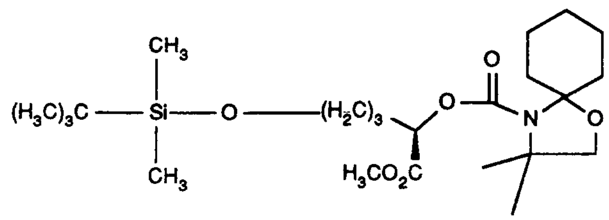

Zu einer Lösung von 692 mg (3,0 mmol) (-)-Spartein in 8 ml wasserfreiem Ether werden bei -78 °C 2,9 mmol sec.-BuLi (in Cyclohexan/Isopentan) gegeben. Nach 10 min Rühren wird die Lösung von 795 mg (2,0 mmol) der Verbindung aus Beispiel XIII in 2 ml Ether injiziert und 6 h bei -78 °C gerührt. Nach der Metallisierung wird trockenes Kohlendioxid eingeleitet und zur Vervollständigung der Reaktion noch 15 h nachgerührt, wobei sich das Gemisch langsam auf Raumtemperatur erwärmt. Der Inhalt wird auf 10 ml 2 N HCl und 10 ml Ether gegossen, die organische Phase abgetrennt und die wäßrige Phase dreimal mit je 20 ml Ether extrahiert. Die Lösung wird am Rotationsverdampfer auf ein Volumen von 10 ml eingeengt und unter Zusatz von drei Tropfen Methanol so lange mit einer ca. 1,5 M Lösung von Diazometan in Ether versetzt, bis die auf das Diazomethan zurückzuführende gelbe Farbe nicht mehr verschwindet. Nach 7 h Rühren bei Raumtemperatur wird das überschüssige Diazomethan durch Zusatz von wenig Kieselgel vernichtet. Das Reaktionsgemisch wird im Vakuum vom Lösemittel befreit und an Kieselgel (Ether/Pentan 1:3) gereinigt. Es werden 705 mg (77%) der Titelverbindung mit >95% ee als farbloses Öl isoliert.

$R_f$ = 0,55 (Ether/Pentan 1:1)

In Analogie zu der Vorschrift des Beispiels 32 werden die in Tabelle 3 aufgeführten Verbindungen hergestellt.

Tabelle 3:

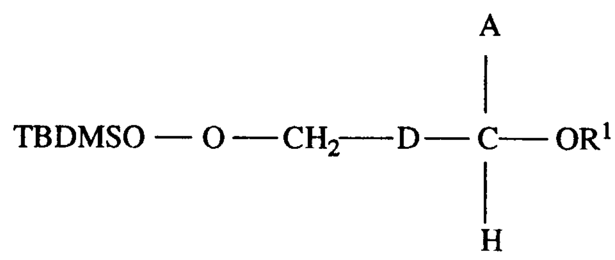

| Bsp.-Nr. | D | A | $R^1$ | Base/Amin | Ausbeute (% d.Th.) | Schmp. (°C) | Konfiguration / ee (%) |
|---|---|---|---|---|---|---|---|
| 33 | $-CH_2-$ | $-CH_3$ | b | s-BuLi/(-)-Sp. | 87 | Öl | S / >97 |
| 34 | $-(CH_2)_2-$ | $-CH_3$ | a | s-BuLi/(-)-Sp. | 81 | Öl | S / >98 |
| 35 | $-(CH_2)_2-$ | $-CH_3$ | a | s-BuLi/TMEDA | 67 | Öl | Racemat |
| 36 | $-(CH_2)_2-$ | $-(CH_3)_3Si$ | a | s-BuLi/(-)-Sp. | 71 | Öl | S / 94 |
| 37 | $-(CH_2)_2-$ | $-(CH_3)_3Si$ | a | s-BuLi/TMEDA | 77 | Öl | Racemat |
| 38 | $-(CH_2)_2-$ | $-CO_2CH_3$ | a | s-BuLi/(-)-Sp. | 77 | Öl | R |
| 39 | $-(CH_2)_2-$ | $-CO_2CH_3$ | a | s-BuLi/TMEDA | 92 | Öl | Racemat |
| 40 | $-(CH_2)_{10}-$ | $-CH_3$ | b | s-BuLi/(-)-Sp. | | Öl | S |

Tabelle 4

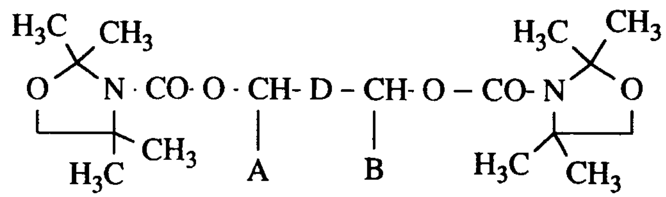

| Bsp. Nr. | A | B | D | F°C/$R_f$* | Ausbeute (% d.Th.) | Drehwert $[\alpha]_D^{20}$ | Base/Komplexbildner | Elektrophil | Ausgangsverbindung (geg.Bsp.Nr.) |
|---|---|---|---|---|---|---|---|---|---|
| 41 | H | $Sn(CH_3)_3$ | $-CH_2$ | | | | | | |
| 42 | H | $(CH_2)_5-CH_3$ | $-CH_2$ | Öl, 0,65[1)] | 33 | +12,17 (C=1,2/Aceton) | BuLi | $J-(CH_2)_5CH_3$ | 41 |
| 43 | H | $CH_3$ | $-CH(CH_2)_3CH_3$ | Öl, 0,50[1)] | 52 | | sec.BuLi/ (-)-Spartein | $CH_3-J$ | XIX |
| 44 | $(CH_3)_3Sn$ | $CH_3$ | $-CH_2$ | Öl, 0,61[1)] | 42 | -16,8 (C=1/Aceton) | sec.BuLi/ TMEDA | $(CH_3)_3SnCl$ | 1 |
| 45 | $(CH_3)_3Sn$ | $CH_3$ | $-CH_2$ | Öl, 0,58[1)] | 50 | +34,8 (C=1,5/Aceton) | sec.BuLi/ (-)-Spartein | $(CH_3)_3SnCl$ | 1 |
| 46 | $(CH_3)_3Si$ | $CH_3$ | $-CH_2$ | Öl, 0,54[1)] | 20 | +25,23 (C=0,75/Aceton) | sec.BuLi/ (-)-Spartein | $(CH_3)_3SiCl$ | 1 |

Tabelle 4 (Fortsetzung)

| Bsp. Nr. | A | B | D | F°C/$R_f$* | Ausbeute (% d.Th.) | Drehwert $[\alpha]_D^{20}$ | Base/Komplexbildner | Elektrophil | Ausgangsverbindung (geg.Bsp.Nr.) |
|---|---|---|---|---|---|---|---|---|---|
| 47 | H | $Sn(CH_3)_3$ | $-CH_2$ | | | | | | |
| 48 | H | $CH_2-CH_3$ | $-CH_2$ | Öl, 0,42[1)] | 61 | +19,6 (C=1/Aceton) | n-Buli DME | $C_2H_5-J$ | 47 |
| 49 | H | OH<br>$C(C_6H_5)_2$ | $-CH_2$ | Öl, 0,32[1)] | 79 | +53,6 (C=1/Aceton) | n-Buli | $(C_6H_5)_2C=0$ | 47 |
| 50 | H | $Sn(CH_3)_3$ | $-CH_2$ | Öl, 0,60[1)] | 98 | +33,6 (C=1/Aceton) | sec-Buli | $(CH_3)_3SnCl$ | V |
| 51 | H | $CH_3$ | $-(CH_2)_2-$ | Öl | | +7,6 (C=1,1, Methylenchlorid) | sec-Buli (-)-Spartein | $CH_3J$ | XX |
| 52 | H | $CO_2CH_3$ | $-CH_2-CH-$<br>$N(CH_2C_6H_5)_2$ | Öl, 0,17[5)] | 82 | -22,57 (C=1,09 Chloroform) | sec-Buli (-)-Spartein | $-CO_2$<br>$CH_3-N_2$ | XVII |
| 53 | $CH_3$ | H | $-CH_2-CH-$<br>$N(CH_2C_6H_5)_2$ | 98-100 | 22 | -12,39 (C=1,77, Chloroform) | sec-Buli (-)-Spartein | $CH_3J$ | XVIII |

Tabelle 4 (Fortsetzung)

| Bsp. Nr. | A | B | D | F°C/$R_f$* | Ausbeute (% d.Th.) | Drehwert $[\alpha]_D^{20}$ | Base/Komplexbildner | Elektrophil | Ausgangsverbindung (geg.Bsp.Nr.) |
|---|---|---|---|---|---|---|---|---|---|
| 54 | H | 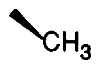 | 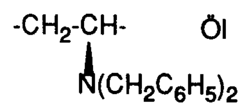 | Öl | 70 | -16,75 (C=1,14 Chloroform) | sec-Buli (-)-Spartein | $CH_3J$ | XVIII |
| 55 | $-Sn(CH_3)_3$ | H | 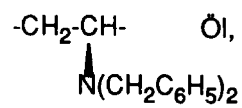 | Öl, 0,39[5)] | | -37,16 (C=2,05, $CHCl_3$) | sec-Buli (-)-Spartein | $Cl-Sn(CH_3)_3$ | XVIII |
| 56 | H | 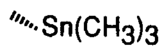 | 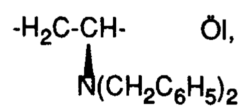 | Öl, 0,31[5)] | | +19,38 (C=0,69, $CHCl_3$) | sec-Buli (-)-Spartein | $Cl-Sn(CH_3)_3$ | XVIII |

Anhand des Beispiels A wird die große Bandbreite des Einsatzes der erfindungsgemäßen Verbindungen beispielsweise gezeigt:

Beispiel A

(2S, 4S)-Pentan-2,4-diol

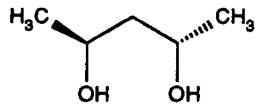

260 mg (0,53 mmol) der Verbindung aus Beispiel 21 werden in 10 ml Methanol gelöst, mit 0,1 ml Methansulfonsäure versetzt und 12 h unter Rückfluß erhitzt. Zur Reaktionsmischung wird 1 g Bariumhydroxid gegeben und weitere 4 h unter Rückfluß erhitzt. Die Reaktionslösung wird vom Lösemittel befreit und das Rohprodukt ohne weitere Aufarbeitung an Kieselgel mit Essigsäureethylester / Hexan 1,5:1 gereinigt. Man erhält 47 mg (85%) der Titelverbindung als farblosen Feststoff.
Schmelzbereich 39-43 °C
$R_f$ = 0,10 (Kieselgel Essigsäureethylester/Hexan 1:1)

**Patentansprüche**

**1.** Dicarbamate der allgemeinen Formel I

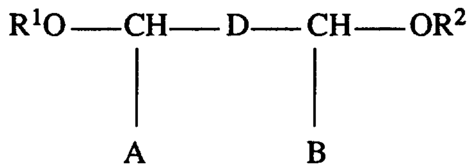

(I)

in welcher

| | |
|---|---|
| $R^1$ und $R^2$ | gleich oder verschieden sind und für einen heterocyclischen Rest der Formel 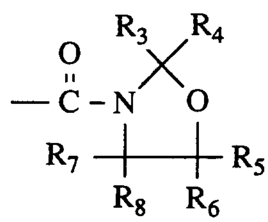 stehen, worin |
| $R^3$, $R^4$, $R^5$, $R^6$, $R^7$ und $R^8$ | gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten, oder jeweils $R^3$ und $R^4$, $R^5$ und $R^6$ und/oder $R^7$ und $R^8$ gemeinsam einen 3-bis 6-gliedrigen, gesättigten Carbocyclus bilden, oder |
| $R^1$ | für die $(CH_3)_3C$-Si-$(CH_3)_2$-Gruppe steht und |
| $R^2$ | die oben angegebene Bedeutung hat, |
| A und B | gleich oder verschieden sind und für geradkettiges oder verzweigtes Alkyl oder Alkenyl mit jeweils bis zu 10 Kohlenstoffatomen stehen, die gegebenenfalls bis zu 3-fach gleich oder verschieden durch Hydroxy, Phenyl oder Cycloalkyl mit 3 bis 7 Kohlenstoffatomen oder durch eine Grup- |

pe der Formel $-NR^9R^{10}$, $-HN-CO-OR^{11}$, $-SiR^{12}R^{13}R^{14}$ oder $SnR^{12'}R^{13'}R^{14'}$ subsitutiert sind,
worin

| | |
|---|---|
| $R^9$ und $R^{10}$ | gleich oder verschieden sind und Wasserstoff, geradkettiges oder verzweigtes Alkyl mit bis zu 8 Kohlenstoffatomen, Phenyl oder Benzyl bedeuten, und |
| $R^{11}$ | geradkettiges oder verzweigtes Alkyl mit bis zu 6 Kohlenstoffatomen bedeutet, das gegebenenfalls durch Phenyl substituiert ist, |
| $R^{12}$, $R^{12'}$, $R^{13}$, $R^{13'}$, $R^{14}$ und $R^{14'}$ | gleich oder verschieden sind und Wasserstoff, geradkettiges oder verzweigtes Alkyl mit bis zu 8 Kohlenstoffatomen oder Phenyl bedeuten, |
| | für geradkettiges oder verzweigtes Acyl mit bis zu 8 Kohlenstoffatomen stehen, |
| | für Cycloalkyl oder Cycloalkenyl mit 3 bis 7 Kohlenstoffatomen stehen, das gegebenenfalls durch Hydroxy substituiert ist, |
| | für Carboxy, Alkoxycarbonyl mit bis zu 4 Kohlenstoffatomen oder für eine Gruppe der Formel - $SiR^{12}R^{13}R^{14}$, $-SnR^{12'}R^{13'}R^{14'}$ oder $R^{15}$-CO stehen, worin |
| $R^{12}$, $R^{12'}$, $R^{13}$, $R^{13'}$, $R^{14}$ und $R^{14'}$ | die oben angegebene Bedeutung haben und |
| $R^{15}$ | Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH-CO-OR^{11}$ substituiert sind, worin |
| $R^{11}$ | die oben angegebene Bedeutung hat, oder |
| | einer der beiden Substituenten A oder B für Wasserstoff steht, |
| D | für geradkettiges oder verzweigtes Alkyl mit bis zu 15 Kohlenstoffatomen steht, das gegebenenfalls durch Phenyl oder durch die Gruppe der Formel $-NR^9R^{10}$ substituiert ist, worin $R^9$ und $R^{10}$ die oben angegebene Bedeutung haben. |

**2.** Verbindungen der allgemeinen Formel I gemäß Anspruch 1, in welcher
$R^1$ und $R^2$ gleich oder verschieden sind und für einen heterocyclischen Rest der Formel

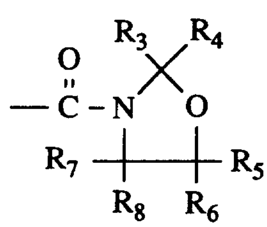

stehen,
worin

| | |
|---|---|
| $R^3$, $R^4$, $R^5$, $R^6$, $R^7$ und $R^8$ | gleich oder verschieden sind und Wasserstoff, geradkettiges oder verzweigtes Alkyl mit bis zu 6 Kohlenstoffatomen, Cyclopropyl, Cyclopentyl oder Cyclohexyl bedeuten, oder $R^3$ und $R^4$, $R^5$ und $R^6$ und/oder $R^7$ und $R^8$ gemeinsam einen Cyclopropyl-, Cyclopentyl- oder Cyclohexylring bilden, oder |
| $R^1$ | für die $(CH_3)_3C-Si-(CH_3)_2$-Gruppe steht und |
| $R^2$ | die oben angegebene Bedeutung hat, |

A und B gleich oder verschieden sind und für geradkettiges oder verzweigtes Alkyl oder Alkenyl mit jeweils bis zu 8 Kohlenstoffatomen stehen, die gegebenenfalls bis zu 3-fach gleich oder verschieden durch Hydroxy, Phenyl, Cyclobutyl, Cyclopentyl, Cyclohexyl oder durch eine Gruppe der Formel $-NR^9R^{10}$, $-NH-CO-OR^{11}$, $-SiR^{12}R^{13}R^{14}$ oder $-SnR^{12'}R^{13'}R^{14'}$ substituiert sind,

worin

$R^9$ und $R^{10}$ gleich oder verschieden sind und Wasserstoff, geradkettiges oder verzweigtes Alkyl mit bis zu 6 Kohlenstoffatomen, Phenyl oder Benzyl bedeuten,

$R^{11}$ geradkettiges oder verzweigtes Alkyl mit bis zu 4 Kohlenstoffatomen bedeutet, das gegebenenfalls durch Phenyl substituiert ist,

$R^{12}$, $R^{12'}$, $R^{13}$, $R^{13'}$, $R^{14}$ und $R^{14'}$ gleich oder verschieden sind und geradkettiges oder verzweigtes Alkyl mit bis zu 4 Kohlenstoffatomen oder Phenyl stehen,

für geradkettiges oder verzweigtes Acyl mit bis zu 6 Kohlenstoffatomen stehen,

für Cyclobutyl oder Cyclohexyl stehen, die gegebenenfalls durch Hydroxy substituiert sind,

für Carboxy, Alkoxycarbonyl mit bis zu 3 Kohlenstoffatomen oder für eine Gruppe der Formel $-SiR^{12}R^{13}R^{14}$, $-SnR^{12'}R^{13'}R^{14'}$ oder $R^{15}-CO-$ steht,

worin

$R^{12}$, $R^{12'}$, $R^{13}$, $R^{13'}$, $R^{14}$ und $R^{14'}$ die oben angegebene Bedeutung haben

und

$R^{15}$ Wasserstoff oder geradkettiges oder verzweigtes Alkyl oder Alkenyl mit jeweils bis zu 6 Kohlenstoffatomen bedeutet, die gegebenenfalls durch Phenyl oder durch die Gruppe der Formel $-NH-CO-OR^{11}$ substituiert sind,

worin

$R^{11}$ die oben angegebene Bedeutung hat,

oder

einer der beiden Substituenten A oder B für Wasserstoff steht,

D für geradkettiges oder verzweigtes Alkyl mit bis zu 14 Kohlenstoffatomen steht, das gegebenenfalls durch Phenyl oder durch die Gruppe der Formel $-NR^9R^{10}$ substituiert ist, worin $R^9$ und $R^{10}$ die oben angegebene Bedeutung haben.

**3.** Verbindungen der allgemeinen Formel I gemäß Anspruch 1, in welcher

$R^1$ und $R^2$ gleich oder verschieden sind und für einen heterocyclischen Rest der Formel

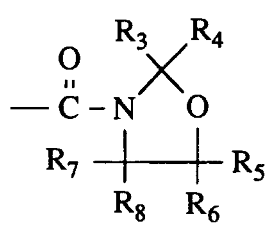

stehen,

worin

$R^3$, $R^4$, $R^5$, $R^6$, $R^7$ und $R^8$ gleich oder verschieden sind und Wasserstoff, geradkettiges oder verzweigtes Alkyl mit bis zu 4 Kohlenstoffatomen, Cyclopropyl oder Cyclopentyl bedeuten, oder $R^3$ und $R^4$, $R^5$ und $R^6$ und/oder $R^7$ und $R^8$ gemeinsam einen Cyclopropyl-, Cyclopentyl-oder Cyclohexylring bilden,

oder

| | |
|---|---|
| $R^1$ | für die $(CH_3)_3C\text{-}Si(CH_3)_2$-Gruppe steht und |
| $R^2$ | die oben angegebene Bedeutung hat, |
| A und B | gleich oder verschieden sind und für geradkettiges oder verzweigtes Alkyl oder Alkenyl mit jeweils bis zu 7 Kohlenstoffatomen stehen, die gegebenenfalls bis zu 3-fach, gleich oder verschieden, durch Hydroxy, Phenyl, Cyclobutyl, Cyclohexyl oder durch eine Gruppe der Formel $-NR^9R^{10}$, - $NHCO\text{-}OR^{11}$, - $SiR^{12}R^{13}R^{14}$ oder $-SnR^{12'}R^{13'}R^{14'}$ substituiert sind worin |
| $R^9$ und $R^{10}$ | gleich oder verschieden sind und Wasserstoff, geradkettiges oder verzweigtes Alkyl mit bis zu 4 Kohlenstoffatomen, Phenyl oder Benzyl bedeuten, |
| $R^{11}$ | Methyl oder Ethyl bedeutet, die gegebenenfalls durch Phenyl substituiert sind, |
| $R^{12}$, $R^{12'}$, $R^{13}$, $R^{13'}$, $R^{14}$ und $R^{14'}$ | für Methyl oder Phenyl stehen, für geradkettiges oder verzweigtes Acyl mit bis zu 4 Kohlenstoffatomen stehen, für Cyclobutyl oder Cyclohexyl stehen, die gegebenenfalls durch Hydroxy substituiert sind, für Carboxy, Methoxycarbonyl mit bis zu 3 Kohlenstoffatomen oder für eine Gruppe der Formel - $SiR^{12}R^{13}R^{14}$, $-SnR^{12'}R^{13'}R^{14'}$ oder $R^{15}$-CO- stehen, worin |
| $R^{12}$, $R^{12'}$, $R^{13}$, $R^{13'}$, $R^{14}$ und $R^{14'}$ | die oben angegebene Bedeutung haben und |
| $R^{15}$ | Wasserstoff oder geradkettiges oder verzweigtes Alkyl oder Alkenyl mit jeweils bis zu 4 Kohlenstoffatomen bedeutet, die gegebenenfalls durch Phenyl oder durch die Gruppe der Formel $-NH\text{-}CO\text{-}OR^{11}$ substituiert sind, worin |
| $R^{11}$ | die oben angegebene Bedeutung hat, oder einer der beiden Substituenten A oder B für Wasserstoff steht, |
| D | für geradkettiges oder verzweigtes Alkyl mit bis zu 12 Kohlenstoffatomen steht, das gegebenenfalls durch Phenyl oder durch die Gruppe der Formel $-NR^9R^{10}$ substituiert ist, worin $R^9$ und $R^{10}$ die oben angegebene Bedeutung haben. |

**4.** Verfahren zur Herstellung von Verbindungen der allgemeinen Formel I

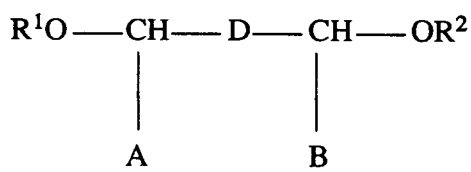

(I)

in welcher

| | |
|---|---|
| $R^1$ und $R^2$ | gleich oder verschieden sind und für einen heterocyclischen Rest der Formel |

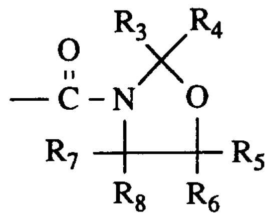

| | |
|---|---|
| | stehen, worin |
| $R^3$, $R^4$, $R^5$, $R^6$, $R^7$ und $R^8$ | gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten, oder jeweils $R^3$ und $R^4$, $R^5$ und $R^6$ und/oder $R^7$ und $R^8$ gemeinsam einen 3-bis 6-gliedrigen, gesättigten Carbocyclus bilden, oder |
| $R^1$ | für die $(CH_3)_3C\text{-}Si\text{-}(CH_3)_2$-Gruppe steht und |
| $R^2$ | die oben angegebene Bedeutung hat, |
| A und B | gleich oder verschieden sind und für geradkettiges oder verzweigtes Alkyl oder Alkenyl mit jeweils bis zu 10 Kohlenstoffatomen stehen, die gegebenenfalls bis zu 3-fach gleich oder verschieden durch Hydroxy, Phenyl oder Cycloalkyl mit 3 bis 7 Kohlenstoffatomen oder durch eine Gruppe der Formel $-NR^9R^{10}$, $-HN\text{-}CO\text{-}OR^{11}$, $-SiR^{12}R^{13}R^{14}$ oder $SnR^{12'}R^{13'}R^{14'}$ subsitutiert sind, worin |
| $R^9$ und $R^{10}$ | gleich oder verschieden sind und Wasserstoff, geradkettiges oder verzweigtes Alkyl mit bis zu 8 Kohlenstoffatomen, Phenyl oder Benzyl bedeuten, und |
| $R^{11}$ | geradkettiges oder verzweigtes Alkyl mit bis zu 6 Kohlenstoffatomen bedeutet, das gegebenenfalls durch Phenyl substituiert ist, |
| $R^{12}$, $R^{12'}$, $R^{13}$, $R^{13'}$, $R^{14}$ und $R^{14'}$ | gleich oder verschieden sind und Wasserstoff, geradkettiges oder verzweigtes Alkyl mit bis zu 8 Kohlenstoffatomen oder Phenyl bedeuten, für geradkettiges oder verzweigtes Acyl mit bis zu 8 Kohlenstoffatomen stehen, für Cycloalkyl oder Cycloalkenyl mit 3 bis 7 Kohlenstoffatomen stehen, das gegebenenfalls durch Hydroxy substituiert ist, für Carboxy, Alkoxycarbonyl mit bis zu 4 Kohlenstoffatomen oder für eine Gruppe der Formel - $SiR^{12}R^{13}R^{14}$, $-SnR^{12'}R^{13'}R^{14'}$ oder $R^{15}$-CO stehen, worin |
| $R^{12}$, $R^{12'}$, $R^{13}$, $R^{13'}$, $R^{14}$ und $R^{14'}$ | die oben angegebene Bedeutung haben und |
| $R^{15}$ | Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH\text{-}CO\text{-}OR^{11}$ substituiert sind, worin |
| $R^{11}$ | die oben angegebene Bedeutung hat, oder einer der beiden Substituenten A oder B für Wasserstoff steht, |
| D | für geradkettiges oder verzweigtes Alkyl mit bis zu 15 Kohlenstoffatomen steht, das gegebenenfalls durch Phenyl oder durch die Gruppe der Formel $-NR^9R^{10}$ substituiert ist, worin $R^9$ und $R^{10}$ die oben angegebene Bedeutung haben, |

dadurch gekennzeichnet, daß man Verbindungen der allgemeinen Formel II

$R^1O\text{-}H_2C\text{-}D\text{-}H_2C\text{-}OR^2$ (II)

in welcher

$R^1$, $R^2$ und D die oben angegebene Bedeutung haben,

in inerten Lösemitteln, in Anwesenheit einer selektiven Base und eines chelatbildenden Diamins (im folgenden mit E bezeichnet) zunächst zu den Carbanion-Komplexverbindungen der allgemeinen Formel (III)

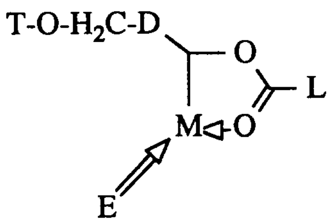

(III)

in welcher

D die oben angegebene Bedeutung hat

und

T für einen der oben aufgeführten Substituenten $R^1$ oder $R^2$ steht,

E für ein chelatbildendes Diamin steht,

M für ein Lithium-, Magnesium-, Titan-, Zirkonium- oder Kaliumatom steht

und

L für den heterocyclischen Rest

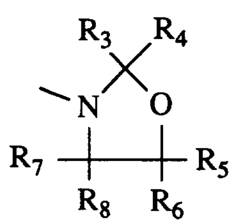

steht,

worin

$R^3$, $R^4$, $R^5$, $R^6$, $R^7$ und $R^8$ die oben angegebene Bedeutung haben,

enantioselektiv deprotoniert; anschließend mit Elektrophilen der allgemeinen Formeln (IV) oder (V)

W-X (IV)

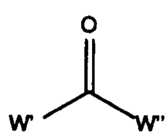

(V)

in welcher

W für einen der oben aufgeführten Substituenten A oder B steht,

X für Halogen, für $C_1$-$C_4$-Alkoxy oder für eine typische Abgangsgruppe steht,

und

W' und W'' gleich oder verschieden sind und für einen unter A aufgeführten chemisch sinnvollen

Rest stehen,
oder mit $CO_2$ in die Verbindungen der allgemeinen Formel (Ia)

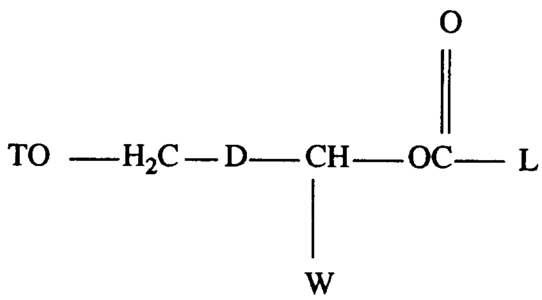 (Ia)

in welcher
T, W, L und D die oben angegebene Bedeutung haben,
umsetzt und im Fall, daß $R^1 \neq (CH_3)_3C\text{-}Si(CH_3)_2$, gegebenenfalls dieses Verfahren zur Einführung des zweiten Substituenten (A oder B) über den Carbanion-Komplex der Formel (VI)

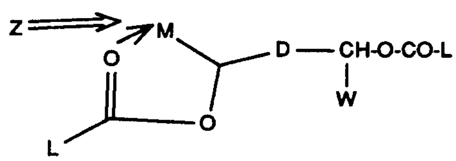 (VI)

in welcher
M, L und W die oben angegebene Bedeutung haben
und

Z entweder für den chiralen Komplexbildner (-)-Spartein oder für den achiralen Komplexbildner TMEDA steht,
unter stereochemischer Lenkung wiederholt.

**5.** Verbindungen der allgemeinen Formel II

$R^1O\text{-}H_2C\text{-}D\text{-}H_2C\text{-}OR^2$ (II)

in welcher
$R^1$, $R^2$ und D die in Anspruch 4 angegebene Bedeutung haben.

**6.** Verfahren zur Herstellung von Verbindungen der allgemeinen Formel II

$R^1O\text{-}H_2C\text{-}D\text{-}H_2C\text{-}OR^2$ (II)

in welcher
$R^1$, $R^2$ und D die in Anspruch 4 angegebene Bedeutung haben,
dadurch gekennzeichnet, daß man Verbindungen der allgemeinen Formel VI

$HO\text{-}H_2C\text{-}D\text{-}CH_2\text{-}OH$ (VI)

in welcher
D die oben angegebene Bedeutung hat,
mit aktivierten Heterocyclen der allgemeinen Formel (VII)

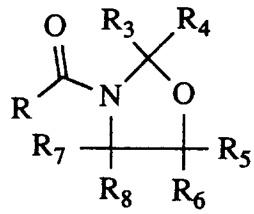

(VII)

in welcher
$R^3$, $R^4$, $R^5$, $R^6$, $R^7$ und $R^8$ die in Anspruch 4 angegebene Bedeutung haben
und
R für Halogen steht,
in einem inerten organischen Lösemittel, gegebenenfalls in Anwesenheit einer Base umsetzt.

**7.** Verbindungen der allgemeinen Formel (Ia)

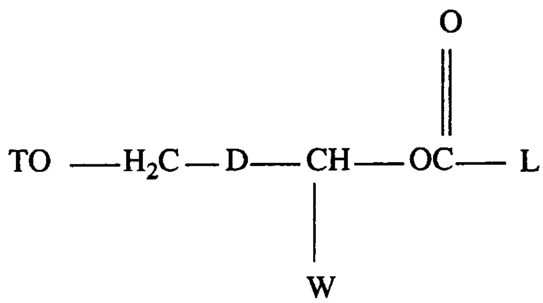

(Ia)

in welcher
T, D, W und L die in Anspruch 4 angegebene Bedeutung haben.

**8.** Verwendung der Verbindungen der allgemeinen Formel (I) und (Ia)gemäß Anspruch 4 bei der Herstellung von 1-Hydroxy-substituierten Cycloalkylverbindungen die für die Synthese von biologisch aktiven Peptiden und Peptid-Mimetika eingesetzt werden können.

Europäisches Patentamt

# EUROPÄISCHER RECHERCHENBERICHT

Nummer der Anmeldung

EP 92 12 0944

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-2 920 075 (SIDNEY MELAMED)<br>* das ganze Dokument *<br>--- | 1 | C07D263/04<br>C07D263/52<br>C07F7/18 |
| A | ANGEWANDTE CHEMIE. INTERNATIONAL EDITION<br>Bd. 29, Nr. 11, November 1990, WEINHEIM DE<br>Seiten 1422 - 1424<br>DIETER HOPPE ET AL 'Chiral lithium-1-oxyalkanides by asymmetric deprotonation;Enantioselective synthesis of 2-hydroxyalkanoic acids and secondary alkanols'<br>* das ganze Dokument *<br>--- | 1-7 | |
| P,X | TETRAHEDRON LETTERS.<br>Bd. 33, Nr. 37, 8. September 1992, OXFORD GB<br>Seiten 5327 - 5330<br>HARTMUT AHRENS ET AL 'Stereoselective generation of 1,3- and 1,4-dioxy-substituted carbanions by sparteine-assisted deprotonation of chiral precursors:subsrate or reagent control in the synthesis of alpha,gamma- and alpha,delta-diols'<br>* das ganze Dokument *<br>--- | 1-7 | RECHERCHIERTE SACHGEBIETE (Int. Cl.5 )<br>C07D<br>C07F |
| P,A | SYNLETT<br>Nr. 9, September 1992, STUTTGART DE<br>Seiten 764 - 766<br>PETRA SOMMMERFELD ET AL 'Enantioselective generation of 3-amino-1-oxy-substituted carbanions by sparteine-induced-deprotonation:asymmetric synthesis of 3-hydroxyalkylamines'<br>* das ganze Dokument *<br>--- | 1-7 | |
| P,A | EP-A-0 464 439 (BAYER AG)<br>* das ganze Dokument *<br>----- | 1-7 | |

Der vorliegende Recherchenbericht wurde für alle Patentansprüche erstellt

| Recherchenort | Abschlußdatum der Recherche | Prüfer |
|---|---|---|
| DEN HAAG | 25 FEBRUAR 1993 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument